# EUROPEAN PATENT SPECIFICATION

(11) **EP 3 177 635 B1**
(45) Date of publication and mention of the grant of the patent: **03.10.2018**
(21) Application number: 15745209.5
(22) Date of filing: 04.08.2015
(51) Int. Cl.: C07K 1/00, C07K 1/02, C07K 1/04, C07K 1/06, C07K 7/16

(54) **PROCESSES FOR THE PREPARATION OF OXYTOCIN ANALOGUES**
VERFAHREN ZUR HERSTELLUNG VON OXYTOCIN-ANALOGA
PROCÉDÉS POUR LA PRÉPARATION D'ANALOGUES DE L'OXYTOCINE

(30) Priority: 07.08.2014 EP 14180161
(43) Date of publication of application: 14.06.2017
(73) Proprietor: F. Hoffmann-La Roche AG, 4070 Basel (CH)
(72) Inventor: BLEICHER, Konrad, 79102 Freiburg (DE); CUENI, Anton, CH-4225 Brislach (CH); PUENTENER, Kurt, 5028 Ueken (CH); SHIINA, Junichi, Kamagura Kanagawa 247-8530 (JP)
(74) Representative: Rauber, Beat
(86) International application number: PCT/EP2015/067881
(87) International publication number: WO 2016/020349

(56) References cited:
- EP-A1- 0 672 680
- WO-A1-2014/095773
- SATOE HASE ET AL: "Effect of an Amide Group in Place of the Disulfide Bridge in Deamino-oxytocin.", J. MED. CHEM., vol. 15, no. 10, 1 January 1972 (1972-01-01), pages 1017-1019, XP55214832, DOI: 10.1021/jm00280a005

## Description

The invention relates to a new process for the preparation of Oxytocin analogues of formula I 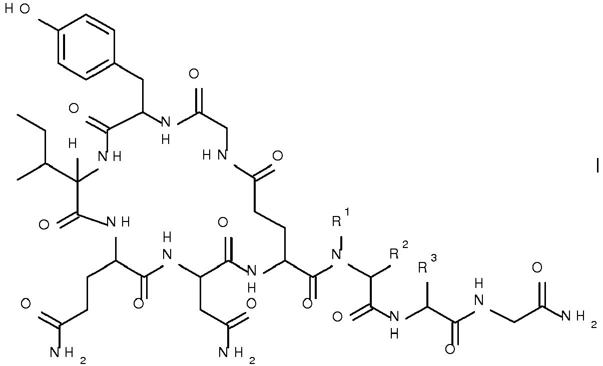 wherein
R¹ is hydrogen or C₁₋₇-alkyl and
R² is hydrogen or C₁₋₇-alkyl; or
R¹ and R² together with the nitrogen and the carbon atom to which they are attached form a 5-membered heterocyle which is optionally substituted with hydroxy or halogen;
R³ is C₁₋₇-alkyl
and its corresponding enantiomers and/ or optical isomers thereof.

Oxytocin analogues of the formula I act as oxytocin receptor agonists and have the potential to be used for the treatment of neurological disorders such as autism, stress, including post-traumatic stress disorder, anxiety, including anxiety disorders and depression, schizophrenia, psychiatric disorders and memory loss, alcohol withdrawal, drug addiction and for the treatment of the Prader-Willi Syndrome (PCT Publication WO 2014/095773).

The preparation of the oxytocin analogues according to process described in the PCT Publication WO 2014/095773 is characterized by the following steps:
x¹) cleavage of Fmoc from a resin bound peptide precursor of the formula X 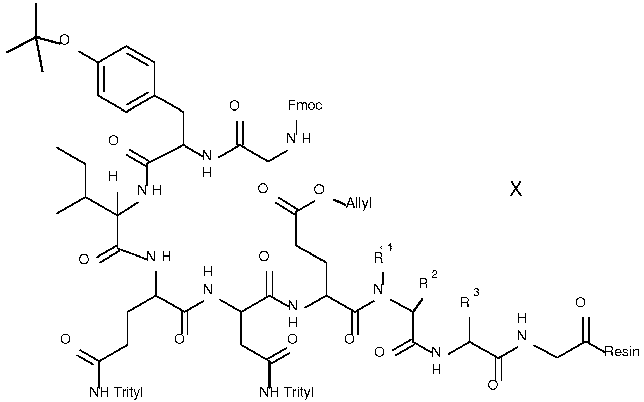
x²) cleavage of the allyl group in a subsequent step
x³) ring cyclization on the resin
x⁴) global deprotection and cleavage from the resin
x⁵) purification and isolation.

It was found that this process known in the art suffers from low overall yields and product selectivity.

Object of the present invention therefore was to improve the synthesis regarding yield and selectivity of the desired Oxytocin analogues.

The object could be achieved with the process of the present invention as outlined hereinafter below.

The process for the preparation of Oxytocin analogues of the formula I 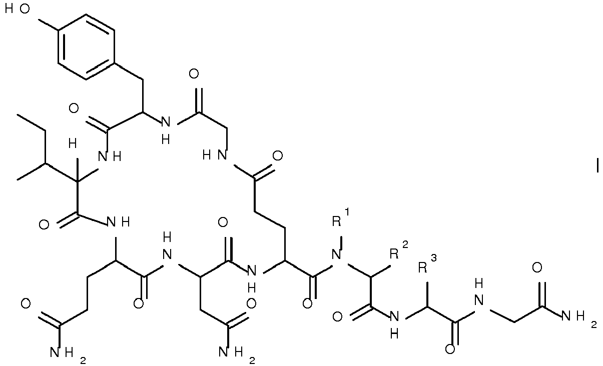 wherein
R¹ is hydrogen or C₁₋₇-alkyl and
R² is hydrogen or C₁₋₇-alkyl; or
R¹ and R² together with the nitrogen and the carbon atom to which they are attached form a 5-membered heterocyle which is optionally substituted with hydroxy or halogen;
R³ is C₁₋₇-alkyl
and of its corresponding enantiomers and/ or optical isomers thereof comprises treating a resin bound peptide precursor of the formula II 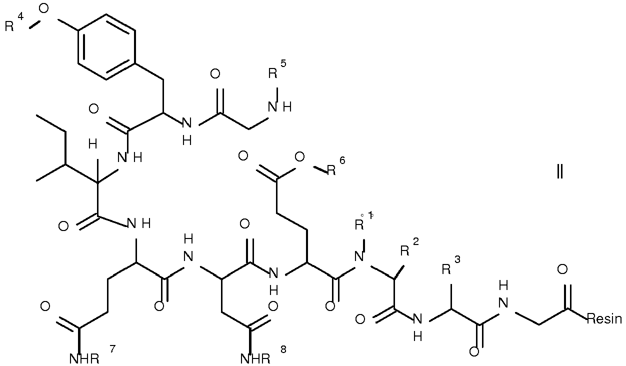 wherein
R¹, R² and R³ are as above and
R⁴ is a hydroxy protecting group;
R⁵ is Fmoc;
R⁶ is t-butyl, 1-adamantyl, or phenylisopropyl;
R⁷ is an amide protecting group; and
R⁸ is an amide protecting group
and its corresponding enantiomers and/ or optical isomers thereof, according to the method:
b) wherein R⁶ is t-butyl, 1-adamantyl or phenylisopropyl and steps b¹), b²), b³) and b⁴) are defined by the appended claims.

The following definitions are set forth to illustrate and define the meaning and scope of the various terms used to describe the invention herein.

The term "C₁₋₇-alkyl" relates to a branched or straight-chain monovalent saturated aliphatic hydrocarbon radical of one to seven carbon atoms, preferably one to four, more preferably one to two carbon atoms. This term is further exemplified by radicals as methyl, ethyl, n-propyl, i-propyl, n-butyl, s-butyl, i-butyl, or t-butyl, pentyl and its isomers, hexyl and its isomers and heptyl and its isomers.

Likewise the term "C₁₋₄-alkyl" relates to a branched or straight-chain monovalent saturated aliphatic hydrocarbon radical of one to four carbon atoms, with the preferences and the respective examples mentioned above.

The term "C₁₋₄-alkyloxy" relates to C₁₋₄-alkyl chain attached to an oxygen atom. This term is further exemplified by radicals as methoxy, ethoxy, n-propoxy, i-propoxy, n-butoxy, i-butoxy and t-butoxy.

The term "C₁₋₄-alkyloxycarbonyl" relates to a C₁₋₄-alkoxy chain attached to a carbonyl group and is further exemplified by the particular alkoxy radicals outlined above attached to a carbonyl group.

The term "C₂₋₄-alkenyl" relates to an unsaturated straight- or branched-carbon chain containing from 2 to 4 carbon atoms containing at least one double bond. This term is further exemplified by radicals as vinyl, allyl and butenyl and its isomers.

The term "halogen" refers to fluorine, chlorine, bromine or iodine.

The term "5-membered heterocyle" which is formed together with R¹ and R² with the nitrogen and the carbon atom to which they are attached stands for a pyrrolidine ring optionally substituted with hydroxy or halogen, particularly for the pyrrolidine ring of proline which is substituted by hydroxy or fluorine.

The term "amide protecting group" refers to an acid or Lewis acid sensitive substituent conventionally used to hinder the reactivity of the amide group. Suitable acid or Lewis acid sensitive amide protecting groups are described in Isidro-Llobet A., Alvarez, M. and Albericio F., "Amino Acid-Protecting Groups", Chem. Rev. 2009, 109, 2455-2504., Chan W. C. and White P. D. "Fmoc Solid Phase Peptide Synthesis", Oxford University Press and Green T., "Protective Groups in Organic Synthesis", 4th Ed. by Wiley Interscience, 2007, Chapter 7, 696 ff.. Suitable amide protecting groups can therefore be selected from trityl, Tmob (2,4,6-trimethoxybenzyl), Xan (9-xanthenyl), Cpd (cyclopropyldimethylcarbinyl), Mbh (4,4'-dimethoxybenzhydryl) or Mtt (4-methyltrityl),

The term "hydroxy protecting group" used for substituent R⁴ refers to any substituents conventionally used to hinder the reactivity of the hydroxy group. Suitable hydroxy protecting groups are described in Isidro-Llobet A., Alvarez, M. and Albericio F., "Amino Acid-Protecting Groups", Chem. Rev. 2009, 109, 2455-2504., Chan W. C. and White P. D. "Fmoc Solid Phase Peptide Synthesis", Oxford University Press, Green T., "Protective Groups in Organic Synthesis", Chapter 1, John Wiley and Sons, Inc.,1991, 10-142 and can be selected from C₁₋₄-alkyl which is optionally substituted with phenyl or halogenated phenyl; C₂₋₄-alkenyl; silyl which is optionally substituted with C₁₋₄-alkyl or phenyl or C₁₋₄-alkyloxycarbonyl.

The spiral bond " 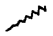 "
stands for " 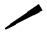 " or for " 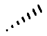 " thus indicating chirality of the molecule.

Whenever a chiral carbon is present in a chemical structure, it is intended that all stereoisomers associated with that chiral carbon are encompassed by the structure as pure stereoisomers as well as mixtures thereof.

In a particular embodiment of the present invention the Oxytocin analogues have the formula Ia 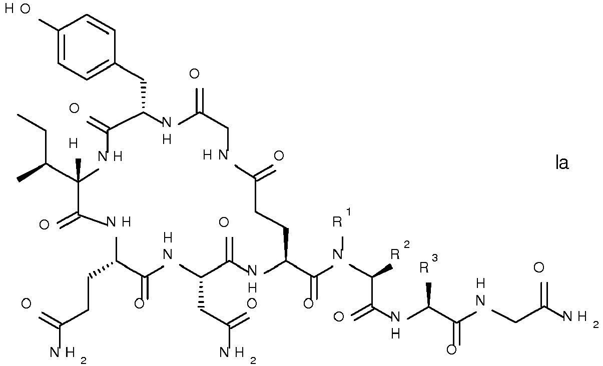 wherein R¹, R² and R³ are as above.
R¹ is particularly hydrogen or C₁₋₄-alkyl, more particularly hydrogen or methyl.
R² is particularly hydrogen or C₁₋₄-alkyl, more particularly hydrogen.
R¹ and R² together with the nitrogen and the carbon atom to which they are attached particularly form the pyrrolidine ring of proline which is optionally substituted with hydroxy or halogen, particularly with hydroxy or fluorine. ;
R³ particularly stands for n-butyl or i-butyl;

Even more particular Oxytocin analogues are listed below:

c[Gly-Tyr-Ile-Gln-Asn-Glu]-Gly-Leu-Gly-NH₂ (1)

c[Gly-Tyr-Ile-Gln-Asn-Glu]-Pro-Leu-Gly-NH₂ (2)

c[Gly-Tyr-Ile-Gln-Asn-Glu]-Sar-Leu-Gly-NH₂ (3)

c[Gly-Tyr-Ile-Gln-Asn-Glu]-Sar-Nle-Gly-NH₂ (4)

c[Gly-Tyr-Ile-Gln-Asn-Glu]-trans-4-fluoro-Pro-Leu-Gly-NH₂ (5)

c[Gly-Tyr-Ile-Gln-Asn-Glu]-trans-4-hydroxy-Pro-Leu-Gly-NH₂ (6).

The resin bound peptide precursor of the formula II has the formula 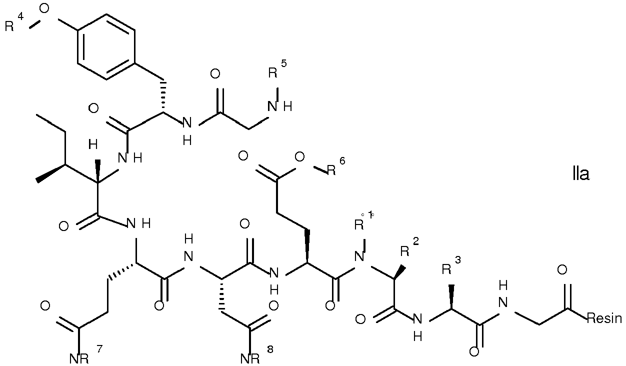 wherein R¹, R², R³, R⁴, R⁵, R⁶, R⁷ and R⁸ are as above.
R¹ is particularly hydrogen or C₁₋₄-alkyl, more particularly hydrogen or methyl.
R² is particularly hydrogen or C₁₋₄-alkyl, more particularly hydrogen.
R¹ and R² together with the nitrogen and the carbon atom to which they are attached particularly form the pyrrolidine ring of proline which is optionally substituted with hydroxy or halogen, particularly with hydroxy or fluorine. ;
R³ particularly stands for n-butyl or i-butyl;
R⁴ particularly is t-butyl, allyl, trityl, 2-chlorotrityl, t-butyloxycarbonyl, t-butyldiphenylsilyl or t-butyldimethylsilyl, but more particularly t-butyl;
R⁵ is Fmoc;
R⁶ particularly is 1-adamantyl, phenylisopropyl or t-butyl,
R⁷ particularly is trityl, 2-chlorotrityl, 4-methyltrityl, but more particularly trityl; and
R⁸ particularly is trityl, 2-chlorotrityl, 4-methyltrityl, but more particularly trityl.

The resin bound peptide precursor of the formula II can be prepared using methods known to the skilled in the art of solid phase peptide synthesis, usually by a repeated Fmoc cleavage and a repeated coupling of the desired Fmoc protected amino acids.

As a rule commercially available amide resins suitable for solid phase peptide synthesis, particularly for Fmoc solid phase peptide synthesis can be used. Useful resins are for instance described in Chan W. C. and White P. D. "Fmoc Solid Phase Peptide Synthesis", Oxford University Press. For example the PL-Rink resin (4-[(2,4-Dimethoxyphenyl)Fmoc-aminomethyl] phenoxyacetamido methyl resin) from Agilent Technology was found to be particular suitable for the process of the present invention.

Fmoc cleavage can happen with a solution of piperidine derivatives in a suitable organic solvent. Advantageously a piperidine or 4-methyl piperidine solution in N,N-dimethylformamide or N-methylpyrrolidone can be applied.

The coupling on the resin with the Fmoc protected amino acids can take place with a coupling agent selected from benzotriazol-1-yl-oxytripyrrolidinophosphonium hexafluorophosphate (PyBOP), (7-azabenzotriazol-1-yloxy)tripyrrolidinophosphonium hexafluorophosphate (PyAOP), bromotripyrrolidinophosphonium hexafluorophosphate (PyBroP), hydroxybenzotriazole (HOBt) and N,N'-diisopropylcarbodiimide (DIC), N,N,N',N'-tetramethyl-O-(benzotriazol-1-yl)-N,N,N',N'-tetramethyluronium hexafluorophosphate (HBTU), O-(7-azabenzotriazol-1-yl)-N,N,N',N'-tetramethyluronium hexafluorophosphate (HATU), O-(6-chlorobenzotriazol-1-yl)-N,N,N',N'-tetramethyluronium hexafluorophosphate (HCTU), (1-cyano-2-ethoxy-2-oxoethylidenaminooxy)dimethylamino-morpholino-carbenium hexafluorophosphate (COMU), tetramethylfluoroformamidinium hexafluorophosphate (TFFH), 2-hydrox-pyridine (HOPy) or 4-(4,6-dimethoxy-1,3,5-triazin-2-yl)-4-methylmorpholinium chloride (DMTMM) in the presence of an organic amine base and a suitable organic solvent.

HOBt, HOPy and DIC in the presence of pyridine as organic amine base and N,N'-dimethlyformamide as organic solvent has been found to be a preferred coupling agent.

The Fmoc-Gly-Tyr(tBu)-Ile-Gln(Trt)-Asn(Trt)-Glu(OAll)-Gly-Leu-Gly-resin of formula X 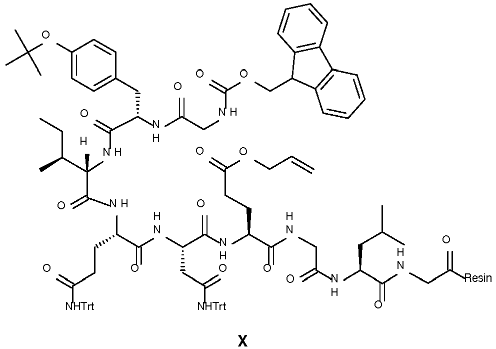 can for instance be built on a PL-Rink resin by repeated Fmoc cleavage and repeated coupling of the following Fmoc-protected amino acids in the order described: Fmoc-Gly-OH, Fmoc-Leu-OH, Fmoc-Gly-OH, Fmoc-Glu(OAll)-OH, Fmoc-Asn(Trt)-OH, Fmoc-Gln(Trt)-OH, Fmoc-Ile-OH, Fmoc-Tyr(tBu)-OH and Fmoc-Gly-OH.

As outline above, the process can follow method a) wherein R⁶ is allyl or 4-{N-[1-(4,4-dimethyl-2,6-dioxocyclohexylidene)-3-methylbutyl]amino}benzyl. In this case the method is characterized by the following steps:
a¹) the allyl or 4-{N-[1-(4,4-dimethyl-2,6-dioxocyclohexylidene)-3-methylbutyl]amino}benzyl group R⁶ is cleaved, in a subsequent step a²) the Fmoc group R⁵ is cleaved, thereafter
a³) ring cyclization is effected on the resin, in a further step
a⁴) global deprotection and cleavage from the resin is effected, and optionally
a⁵) the oxytocin analogue of formula I so obtained is purified and isolated.

The allyl or 4-{N-[1-(4,4-dimethyl-2,6-dioxocyclohexylidene)-3-methylbutyl]amino}benzyl group cleavage in step a¹) is usually performed in presence of a palladium or a rhodium compound or of hydrazine. Suitable palladium or rhodium compounds can be selected from tetrakis(triphenylphosphine) palladium, palladium acetate/triphenylphosphine, palladium acetate/triethylphosphite, bis(triphenylphosphine)palladium dichloride or tris(triphenylphosphine)rhodium chloride. Preferably palladium compounds, even more preferably tetrakis(triphenylphosphine) palladium are used.

In addition a scavenger such as phenylsilane, pyrrolidine, morpholine or N-methyl-N-trimethylsilyl-trifluoroacetamide, particularly phenylsilane is usually present.

The reaction as a rule can happen at room temperature in a suitable organic solvent such as methylene chloride, acetonitrile or tetrahydrofuran.

The Fmoc cleavage in step a²) can be performed as outlined above with piperidine or 4-methyl-piperidine in a suitable organic solvent.

The ring cyclization in step a³) is effected on the resin, expediently using a cyclization agent selected from benzotriazol-1-yl-oxytripyrrolidinophosphonium hexafluorophosphate (PyBOP), (7-azabenzotriazol-1-yloxy)tripyrrolidinophosphonium hexafluorophosphate (PyAOP), N,N,N',N'-tetramethyl-O-(1H-benzotriazol-1-yl)uranium hexafluorophosphate (HBTU), 1-[bis(dimethylamino)methylene]-1H-1,2,3-triazolo[4,5-b]pyridinium 3-oxid hexafluorophosphate (HATU), O-(6-chlorobenzotriazol-1-yl)-N,N,N',N'-tetramethyluronium hexafluorophosphate (HCTU), (1-cyano-2-ethoxy-2-oxoethylidenaminooxy)dimethylamino-morpholino-carbenium hexafluorophosphate (COMU), 2-hydroxy-pyridine (HOPy) or 4-(4,6-dimethoxy-1,3,5-triazin-2-yl)-4-methylmorpholinium chloride (DMTMM) in the presence of an organic amine base.

Suitable organic amine bases can be selected from pyridine, imidazole, N,N-diisopropylethyl amine, triethylamine, N-methylmorpholine, N,N-dimethyl-4-aminopyridine, 1,8-Diazabicyclo[5.4.0]undec-7-ene or 1,4-diazabicyclo[2.2.2]octane.

The cyclization step a³) can be performed with PyBOP or PyAOP in the presence of N, N-diisopropylethyl amine, imidazole or N-methylmorpholine as organic amine bases at temperatures between 0 °C to 25 °C.

Global deprotection and cleavage from the resin in step a⁴) can be effected in the presence of trifluoroacetic acid/water and a suitable scavenger such as thioanisole, anisole, phenol, triisopropylsilane, triethylsilane, ethanedithiol or dithiothreitol usually at temperatures between of 0 °C to 25 °C. Triisopropylsilane has been found to be a preferred scavenger.

In step a⁵) the crude oxytocin analogue can be isolated by filtering off the resin, by removing the solvent from the filtrate and further by taking the residue up in a suitable organic solvent such as in methyl t-butyl ether, 2-methyltetrahydrofuran or in mixtures thereof and by final filtration and drying.

The crude oxytocin analogue can be further purified by preparative HPLC in solution with a suitable organic solvent such as with aqueous acetonitrile and suitable additives such as trifluoroacetic acid, acetic acid or ammonium acetate.

The fractions obtained can then be lyophilized to obtain pure oxytocin analogue of formula I.

The process of the present invention follows method b) wherein R⁶ is t-butyl, 1-adamantyl or phenylisopropyl. In this case the method is characterized by the steps defined by the appended claims.

The Fmoc cleavage in step b¹) can take place as described for step a²) above.

Global deprotection and cleavage from the resin in step b²) can be performed as described above in step a⁴). The preferred embodiments described for step a⁴ likewise apply for step b²).

The ring cyclization in step b³) is effected in solution but can happen with the cyclization agents and the organic amine bases listed for step a³) above. The preferred embodiments described for step a³ likewise apply for step b³).

Isolation and purification in step b⁴) can take place in the same manner as described in step a⁵). The preferred embodiments described for step a⁵ likewise apply for step b⁴).

### Examples

### Abbreviations:

SPPS = Solid-phase peptide synthesis, PL-Rink resin = 4-[(2,4-Dimethoxyphenyl)Fmoc-aminomethyl]phenoxyacetamido methyl resin from Agilent Technology (PL1467-4749: 0.32 mmol/g 75 - 150-10⁻⁶m; PL1467-4799: 0.55 mmol/g 75-150 - 10⁻⁶m; PL1467-4689: 0.96 mmol/g 150-300 - 10⁻⁶m), Fmoc = 9-Fluorenylmethoxycarbonyl, Gly = Glycine, Leu = Leucine, Glu(OAll) = Allyl-protected glutamic acid, Glu(tBu) = tert Butyl-protected glutamic acid, Asn(Trt) = Trityl-protected asparagine, Gln(Trt) = Trityl-protected glutamine, Ile = Isoleucine, Tyr(tBu) = tert Butyl-protected tyrosine, Sar = N-methylglycine, Pro = Proline, Nle = Norleucine, DMF = N,N-Dimethylformamide, HOBt = 1-Hydroxybenzotriazole, HOPy = 2-hyxroxy-pyridine, DIC = N,N'-Diisopropylcarbodiimide, NEP = N-Ethylpyrrolidone, PyBOP = (Benzotriazol-1-yloxy)tripyrrolidinophosphonium hexafluorophosphate, DIPEA = Diisopropylethyl amine, MeOH = Methanol, CH₂Cl₂ = Dichloromethane, MTBE = Methyl tert-butyl ether, MeTHF = 2-Methyltetrahydrofuran, TFA = Trifluoroacetic acid, MeCN = Acetonitrile, PyAOP = (7-Azabenzotriazol-1-yloxy)tripyrrolidinophosphonium hexafluorophosphate, HBTU = N,N,N',N'-Tetramethyl-O-(1H-benzotriazol-1-yl)uranium hexafluorophosphate, HATU = 1-[Bis(dimethylamino)methylene]-1H-1,2,3-triazolo[4,5-b]pyridinium 3-oxid hexafluorophosphate, HCTU = O-(6-Chlorobenzotriazol-1-yl)-N,N,N',N'-tetramethyluronium hexafluorophosphate, COMU = (1-Cyano-2-ethoxy-2-oxoethylidenaminooxy)dimethylamino-morpholino-carbenium hexafluorophosphate, DMTMM = 4-(4,6-Dimethoxy-1,3,5-triazin-2-yl)-4-methylmorpholinium chloride, NMP = 1-Methyl-2-pyrrolidinone, DMSO = Dimethyl sulfoxide, DMI = 1,3-Dimethyl-2-imidazolidinone, DMPU = 1,3-Dimethyl-3,4,5,6-tetrahydro-2(1H)-pyrimidinone, NMM = N-Methylmorpholine, DMAP = N,N-Dimethyl-4-aminopyridine, DIPEA = N,N-Diisopropylethylamine, DBU = 1,8-Diazabicyclo[5.4.0]undec-7-ene, DABCO = 1,4-Diazabicyclo[2.2.2]octane.

### Comparison Example

A comparative experiment was run for the preparation of

c[Gly-Tyr-Ile-Gln-Asn-Glu]-Gly-Leu-Gly-NH₂ (1)

in analogy to the synthesis description of the WO2014/095773 (Solid phase cyclization) and as outlined in scheme 1 below:

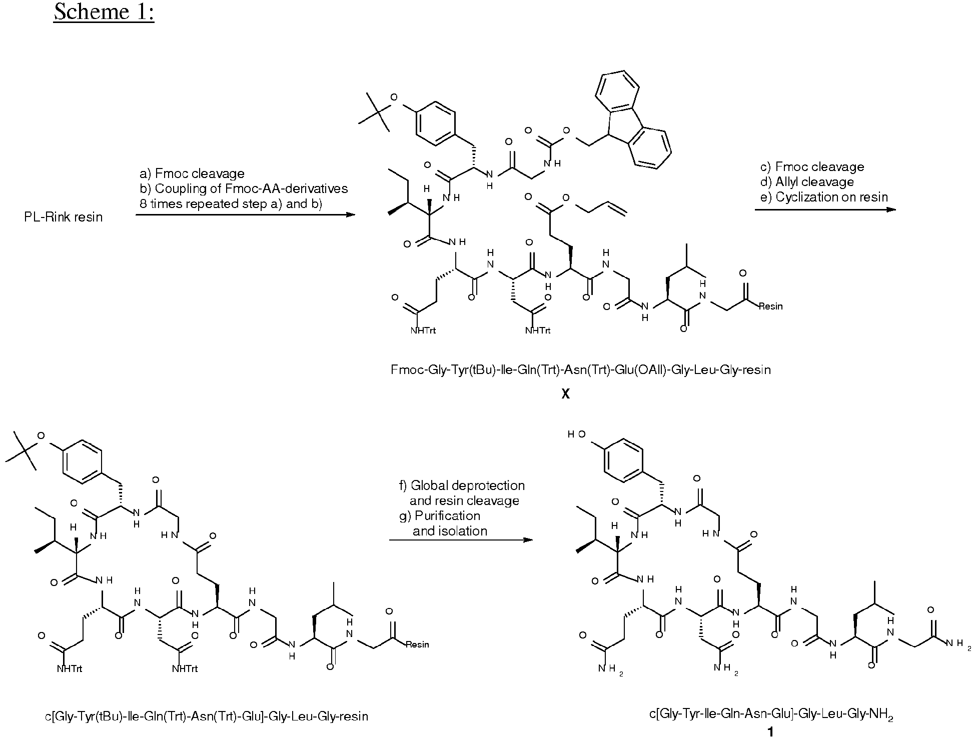

Synthesis performance has been measured based on the yield and the ratio of product (1) to the dimer-by product of the formula shown in scheme 2 below:

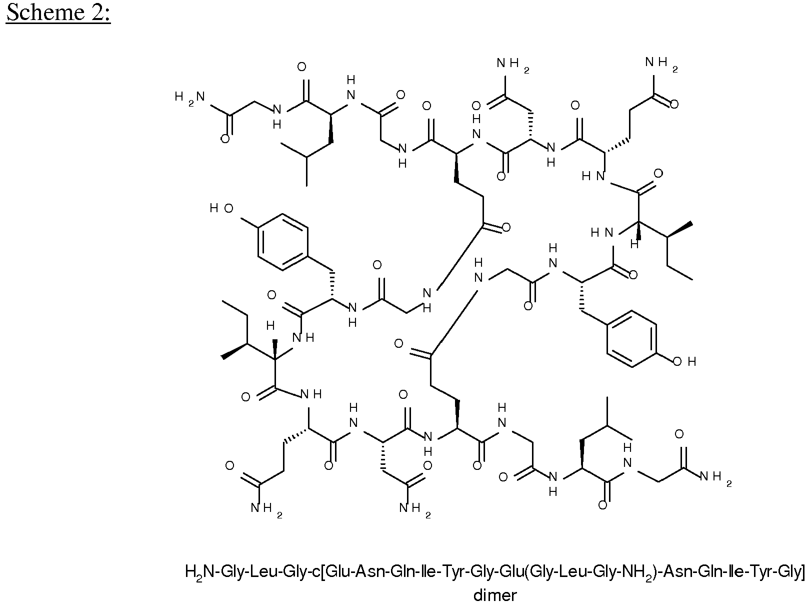

### a) Fmoc-Cleavage:

A SPPS reactor (100 mL; peptide synthesizer CS136XT ex CSBio) was charged with PL-Rink resin (load. 0.55 mmol/g, 5.00 g, 2.75 mmol) and 20% piperidine in DMF (50.0 mL). The mixture was then stirred at 25 °C for 10 min. After draining the solvent, another portion of 20% piperidine in DMF (50.0 mL) was added and the mixture was stirred at 25 °C for 30 min. After draining the solvent, the resultant resin was washed with DMF (8 x 50.0 mL) to yield deFmoc-PL-Rink resin.

### b) Coupling with Fmoc-AA-derivatives:

To deFmoc-PL-Rink resin, a solution of Fmoc-Gly-OH in 0.35M HOBt/DMF (32.0 mL, 11.2 mmol), 0.92M DIC in DMF (16.0 mL, 14.7 mmol) and 10% pyridine in DMF (16.0 mL, 19.8 mmol) were added and stirred at 25 °C for 3 h. After draining the solvent, the resultant resin was washed with DMF (4 x 50.0 mL) to yield Fmoc-Gly-resin.

Fmoc-Cleavage and Fmoc-AA-derivative coupling steps were repeated 8 times employing instead of Fmoc-Gly-OH, the following Fmoc-amino acid-derivatives: Fmoc-Leu-OH, Fmoc-Gly-OH, Fmoc-Glu(OAll)-OH, Fmoc-Asn(Trt)-OH, Fmoc-Gln(Trt)-OH, Fmoc-Ile-OH, Fmoc-Tyr(tBu)-OH, Fmoc-Gly-OH to yield Fmoc-Gly-Tyr(tBu)-Ile-Gln(Trt)-Asn(Trt)-Glu(OAll)-Gly-Leu-Gly-resin. A sample was cleaved from the resin (vide below) to confirm the correct mass. MS (m/z): 1211.8 (M+H)⁺

### c) Fmoc-Cleavage:

Fmoc-Cleavage of the terminal Gly was conducted as described above to yield H-Gly-Tyr(tBu)-Ile-Gln(Trt)-Asn(Trt)-Glu(OAll)-Gly-Leu-Gly-resin. A sample was cleaved from the resin (vehicle below) to confirm the correct mass. MS (m/z): 989.8 (M+H)⁺

### d) Allyl-Cleavage:

To H-Gly-Tyr(tBu)-Ile-Gln(Trt)-Asn(Trt)-Glu(OAll)-Gly-Leu-Gly-resin, a solution of tetrakis triphenylphosphine palladium (159 mg, 0.138 mmol) and phenylsilane (3.40 mL, 27.6 mmol) in CH₂Cl₂ (50.0 mL) was added and stirred at 25 °C for 30 min. After draining the solvent, this step was repeated once more and washed with DMF (2 x 50.0 mL). A solution of sodium dithiocarbamate (250 mg) and DIPEA (0.250 mL) in DMF (50.0 mL) was added and the mixture was stirred at 25 °C for 15 min. After draining the solvent, this step was repeated once more. After draining the solvent, the resultant resin was washed with DMF (4 x 50.0 mL) to yield H-Gly-Tyr(tBu)-Ile-Gln(Trt)-Asn(Trt)-Glu-Gly-Leu-Gly-resin. A sample was cleaved from the resin (vehicle below) to confirm the correct mass. MS (m/z): 949.7 (M+H)⁺

### e) Cyclization on resin:

A solution of PyBOP (2.36 g, 4.54 mmol) and DIPEA (2.40 mL, 13.8 mmol) in NEP (60.0 mL) was added to H-Gly-Tyr(tBu)-Ile-Gln(Trt)-Asn(Trt)-Glu-Gly-Leu-Gly-resin and the mixture was stirred at 25 °C for 4 h. After draining the solvent, the resultant resin was washed with DMF (4 x 50.0 mL), CH₂Cl₂ (3 x 50.0 mL) and MeOH (3 x 50.0 mL). The resin was dried at 10 mbar at 25 °C for 1 day to afford c[Gly-Tyr(tBu)-Ile-Gln(Trt)-Asn(Trt)-Glu]-Gly-Leu-Gly-resin (8.60 g).

### f) Global deprotection and resin cleavage:

To a precooled (10-15 °C) solution of triisopropylsilane (2.80 mL) in TFA (40.0 mL) and water (10.0 mL), c[Gly-Tyr(tBu)-Ile-Gln(Trt)-Asn(Trt)-Glu]-Gly-Leu-Gly-resin (8.60 g) was added and stirred at 25 °C for 3 h. The resin was filtered off and the filtrate was concentrated in vacuo. The residue was added to MTBE (100 mL) and the mixture was stirred at 25 °C for 15 h. The mixture was filtered and the cake was washed with MTBE (50.0 mL) followed by drying to afford crude c[Gly-Tyr-Ile-Gln-Asn-Glu]-Gly-Leu-Gly-NH₂ 1 (2.01 g, assay 11.3 wt%, total 9 % yield) as a white solid with 15.9% purity (HPLC area-%, HPLC method cf. Example 1). The ratio of 1/dimer was 8.5.

**Table 1:**

| Example | Cyclization method | Total yield (%) | Purity of crude cyclic peptide (HPLC area-%) | Ratio of **1**/dimer |
|---|---|---|---|---|
| Comparison | Solid phase | 9 | 15.9 | 8.5 |
| 1 (invention) | Solid phase | 38 | 62.7 | 21.9 |
| 2 (invention) | Liquid phase | 31 | 56.6 | 15.1 |

### Example 1 (Solid phase cyclization)

c[Gly-Tyr-Ile-Gln-Asn-Glu]-Gly-Leu-Gly-NH₂ (1)

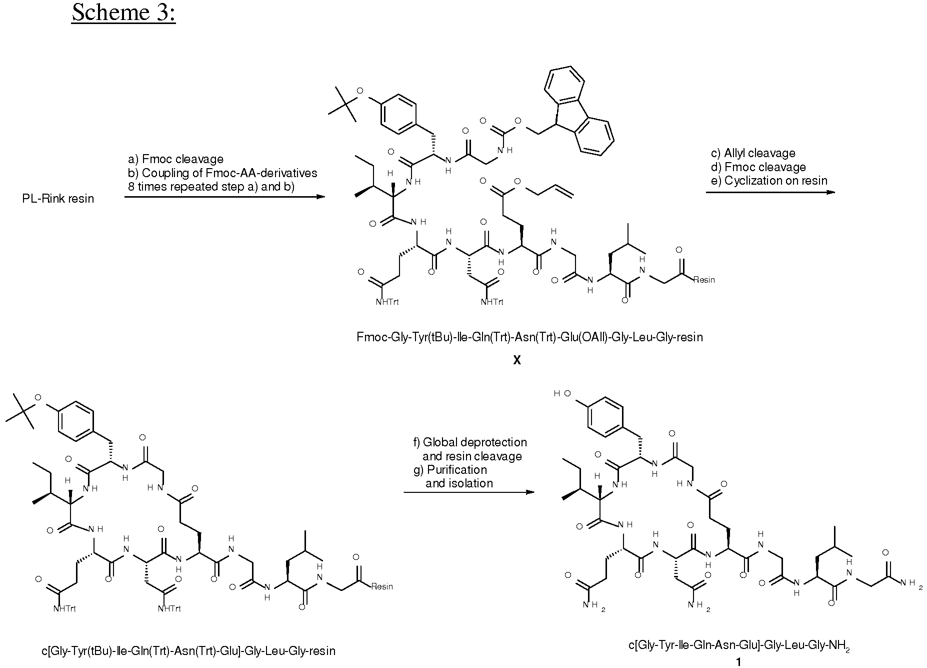

### a) Fmoc-Cleavage:

A SPPS reactor (100 mL; peptide synthesizer CS136XT ex CSBio) was charged with PL-Rink resin (load. 0.55 mmol/g, 5.00 g, 2.75 mmol) and 20% piperidine in DMF (50.0 mL). The mixture was then stirred at 25 °C for 10 min. After draining the solvent, another portion of 20% piperidine in DMF (50.0 mL) was added and the mixture was stirred at 25 °C for 30 min. After draining the solvent, the resultant resin was washed with DMF (8 x 50.0 mL) to yield deFmoc-PL-Rink resin.

### b) Coupling with Fmoc-AA-derivatives:

To deFmoc-PL-Rink resin, a solution of Fmoc-Gly-OH in 0.35M HOBt/DMF (32.0 mL, 11.2 mmol), 0.92M DIC in DMF (16.0 mL, 14.7 mmol) and 10% pyridine in DMF (16.0 mL, 19.8 mmol) were added and stirred at 25 °C for 3 h. After draining the solvent, the resultant resin was washed with DMF (4 x 50.0 mL) to yield Fmoc-Gly-resin.

Fmoc-Cleavage and Fmoc-AA-derivative coupling steps were repeated 8 times employing instead of Fmoc-Gly-OH, the following Fmoc-amino acid-derivatives: Fmoc-Leu-OH, Fmoc-Gly-OH, Fmoc-Glu(OAll)-OH, Fmoc-Asn(Trt)-OH, Fmoc-Gln(Trt)-OH, Fmoc-Ile-OH, Fmoc-Tyr(tBu)-OH, Fmoc-Gly-OH to yield X (Fmoc-Gly-Tyr(tBu)-Ile-Gln(Trt)-Asn(Trt)-Glu(OAll)-Gly-Leu-Gly-resin). A sample was cleaved from the resin (vide below) to confirm the correct mass. MS (m/z): 1211.8 (M+H)⁺

### c) Allyl-Cleavage:

To **X** (Fmoc-Gly-Tyr(tBu)-Ile-Gln(Trt)-Asn(Trt)-Glu(OAll)-Gly-Leu-Gly-resin), a solution of tetrakis triphenylphosphine palladium (159 mg, 0.138 mmol) and phenylsilane (3.40 mL, 27.6 mmol) in CH₂Cl₂ (50.0 mL) was added and stirred at 25 °C for 30 min. After draining the solvent, this step was repeated once more and washed with DMF (2 x 50.0 mL). A solution of sodium dithiocarbamate (250 mg) and DIPEA (0.250 mL) in DMF (50.0 mL) was added and the mixture was stirred at 25 °C for 15 min. After draining the solvent, this step was repeated once more. After draining the solvent, the resultant resin was washed with DMF (4 x 50.0 mL) to yield Fmoc-Gly-Tyr(tBu)-Ile-Gln(Trt)-Asn(Trt)-Glu-Gly-Leu-Gly-resin. A sample was cleaved from the resin (vehicle below) to confirm the correct mass. MS (m/z): 1171.8 (M+H)⁺

### d) Fmoc-Cleavage:

Fmoc-Cleavage of the terminal Gly was conducted as described above to yield H-Gly-Tyr(tBu)-Ile-Gln(Trt)-Asn(Trt)-Glu-Gly-Leu-Gly-resin. A sample was cleaved from the resin (vehicle below) to confirm the correct mass. MS (m/z): 949.7 (M+H)⁺

### e) Cyclization on resin:

A solution of PyBOP (2.36 g, 4.54 mmol) and DIPEA (2.40 mL, 13.8 mmol) in NEP (60.0 mL) was added to (H-Gly-Tyr(tBu)-Ile-Gln(Trt)-Asn(Trt)-Glu-Gly-Leu-Gly-resin and the mixture was stirred at 25 °C for 4 h. After draining the solvent, the resultant resin was washed with DMF (4 x 50.0 mL), CH₂Cl₂ (3 x 50.0 mL) and MeOH (3 x 50.0 mL). The resin was dried at 10 mbar at 25 °C for 1 day to afford c[Gly-Tyr(tBu)-Ile-Gln(Trt)-Asn(Trt)-Glu]-Gly-Leu-Gly-resin (9.17 g).

### f) Global deprotection and resin cleavage:

To a precooled (10-15 °C) solution of triisopropylsilane (2.50 mL) in TFA (40.0 mL) and water (10.0 mL), c[Gly-Tyr(tBu)-Ile-Gln(Trt)-Asn(Trt)-Glu]-Gly-Leu-Gly-resin (9.17 g) was added and stirred at 25 °C for 3 h. The resin was filtered off and the filtrate was concentrated in vacuo. The residue was added to MTBE (100 mL) and the mixture was stirred at 25 °C for 15 h. The mixture was filtered and the cake was washed with MTBE (50.0 mL) followed by drying to afford crude c[Gly-Tyr-Ile-Gln-Asn-Glu]-Gly-Leu-Gly-NH₂ 1 (2.39 g, assay 40.9 wt%, total 38% yield) as a white solid with 62.7% purity (HPLC area-%, HPLC method: Aquity UPLC BEH130 C18 column, 150 x 2.1 mm; mobile phase, A: 0.05% TFA in water, B: 0.05% TFA in MeCN; flow: 0.13 mL/min for 40 min, 0.25 mL/min for 15 min; isocratic 90/10 (A/B) for 3 min, gradient from 90/10 (A/B) to 62/38 (A/B) within 37min, gradient from 62/38 (A/B) to 10/90 (A/B) within 5 min, isocratic 10/90 (A/B) for 10 min. Temp: 60 °C, UV:214nm). The ratio of 1/dimer was 21.9.

Retention time: 23.2 min (c[Gly-Tyr-Ile-Gln-Asn-Glu]-Gly-Leu-Gly-NH₂), 18.8 min (H-Gly-Tyr-Ile-Gln-Asn-Glu-Gly-Leu-Gly-NH₂), 26.1 min (dimer)

### g) Purification and isolation:

Crude c[Gly-Tyr-Ile-Gln-Asn-Glu]-Gly-Leu-Gly-NH₂ was dissolved in water-MeCN (10-1) and filtered. The filtrate was diluted with the same volume of water. The solution was purified by preparative HPLC on a Kromasil-C18-100 column (250 x 80 mm, 10 um particle size, A: 0.1% TFA-water, B: MeCN; flow: 300 mL/min; isocratic 95/5 (A/B) for 2 min, gradient from 95/5 (A/B) to 80/20 (A/B) within 1 min, gradient from 80/20 (A/B) to 77/23 (A/B) within 17min, gradient from 77/23 (A/B) to 10/90 (A/B) within 1 min, isocratic 10/90 (A/B) for 7 min, gradient from 10/90 (A/B) to 95/5 (A/B) within 1 min, isocratic 95/5 (A/B) for 6 min. The fractions were collected and lyophilized to yield pure c[Gly-Tyr-Ile-Gln-Asn-Glu]-Gly-Leu-Gly-NH₂**1** (0.708 g) as a white powder with 99.2% purity (HPLC area-%, HPLC method cf. Example 1). No dimer was observed in pure **1.** MS (m/z): 931.0 (M+H)⁺

### Example 2 (Solution phase cyclization)

c[Gly-Tyr-Ile-Gln-Asn-Glu]-Gly-Leu-Gly-NH₂ (**1**)

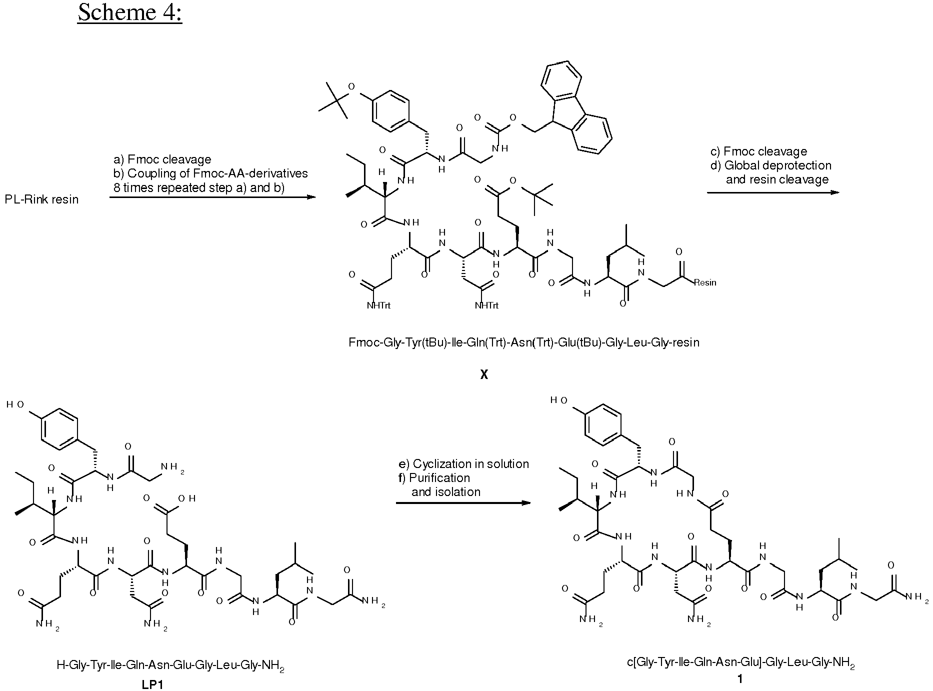

### a) Fmoc-Cleavage:

A SPPS reactor (100 mL) was charged with PL-Rink resin (load. 0.55 mmol/g, 5.00 g, 2.75 mmol) and 20% piperidine in DMF (50 mL). The mixture was then stirred at 25 °C for 10 min. After draining the solvent, another portion of 20% piperidine in DMF (50.0 mL) was added and the mixture was stirred at 25 °C for 30 min. After draining the solvent, the resultant resin was washed with DMF (8 x 50.0 mL) to yield deFmoc-PL-Rink-resin.

### b) Coupling of Fmoc-AA-derivatives:

To deFmoc-PL-Rink-resin, a solution of Fmoc-Gly-OH in 0.35M HOBt/DMF (32.0 mL, 11.2 mmol), 0.92M DIC in DMF (16.0 mL, 14.7 mmol) and 10% pyridine in DMF (16.0 mL, 19.8 mmol) were added and stirred at 25 °C for 3 h. After draining the solvent, the resultant resin was washed with DMF (4 x 50.0 mL) to yield Fmoc-Gly-resin.

Fmoc-Cleavage and Fmoc-AA-derivative coupling steps were repeated 8 times employing instead of Fmoc-Gly-OH, the following Fmoc-amino acid-derivatives: Fmoc-Leu-OH, Fmoc-Gly-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH to yield **X** (Fmoc-Gly-Tyr(tBu)-Ile-Gln(Trt)-Asn(Trt)-Glu(tBu)-Pro-Leu-Gly-resin). A sample was cleaved from the resin (vide below) to confirm the correct mass. MS (m/z): 1171.8 (M+H)⁺

### c) Fmoc-Cleavage:

Fmoc-Cleavage of the terminal Gly was conducted as described above. After draining the solvent, the resultant resin was washed with DMF (8 x 50.0 mL), CH₂Cl₂ (3 x 50.0 mL) and MeOH (3 x 50.0 mL). The resin was dried at 10 mbar at 25 °C for 1 day to afford to yield H-Gly-Tyr(tBu)-Ile-Gln(Trt)-Asn(Trt)-Glu(tBu)-Gly-Leu-Gly-resin (10.8 g).

### d) Global deprotection and resin cleavage:

To a precooled (10-15 °C) solution of triisopropylsilane (2.50 mL) in TFA (40.0 mL) and water (10.0 mL), H-Gly-Tyr(tBu)-Ile-Gln(Trt)-Asn(Trt)-Glu(tBu)-Gly-Leu-Gly-resin (10.8 g) was added and stirred at 25 °C for 3 h. The resin was filtered off and the filtrate was concentrated in vacuo. The residue was added to MeTHF (100 mL) and the mixture was stirred at 25 °C for 15 h. The mixture was filtered and the cake was washed with MeTHF (50.0 mL) followed by drying to afford **LP1** (H-Gly-Tyr-Ile-Gln-Asn-Glu-Gly-Leu-Gly-NH₂) (3.60 g) as a white solid with 67.4% purity (HPLC area-%, HPLC method cf. example 1). MS (m/z): 949.7 (M+H)⁺

### e) Cyclization in solution:

To a mixture of **LP1** (H-Gly-Tyr-Ile-Gln-Asn-Glu-Gly-Leu-Gly-NH₂) (3.50 g) in NEP (60.0 mL) and DIPEA (3.13 mL, 18.4 mmol) was added PyBOP (1.92 g, 3.69 mmol) and stirred at 25 °C for 1 h. To complete conversion, another portion of PyBOP (0.960 g, 1.84 mmol) was added and stirred at the same temperature for 1 h. The resultant mixture was added to a solution of MTBE/MeTHF solution (400 mL/100 mL) and stirred at 25 °C for 15 h. The mixture was filtered and the cake was washed with MTBE (50.0 mL) followed by drying to afford crude c[Gly-Tyr-Leu-Gln-Asn-Glu]-Gly-Leu-Gly-NH₂**1** (4.30 g, assay 18.0 wt%, total 31% yield) as a white solid with 56.6% purity (HPLC area-%, HPLC method cf. example 1). The ratio of **1**/dimer was 15.1.

### f) Purification and isolation:

Crude c[Gly-Tyr-Leu-Gln-Asn-Glu]-Gly-Leu-Gly-NH₂ was dissolved in water-MeCN (10-1) and filtered off undissolved material. The filtrate was diluted with the same volume of water. The solution was purified by preparative HPLC on a Kromasil-C18-100 column (250 x 80 mm, 10 um particle size, A: 0.1% TFA-water, B: MeCN; flow: 300 mL/min; isocratic 95/5 (A/B) for 2 min, gradient from 95/5 (A/B) to 80/20 (A/B) within 1 min, gradient from 80/20 (A/B) to 77/23 (A/B) within 17min, gradient from 77/23 (A/B) to 10/90 (A/B) within 1 min, isocratic 10/90 (A/B) for 7 min, gradient from 10/90 (A/B) to 95/5 (A/B) within 1 min, isocratic 95/5 (A/B) for 6 min. The fractions were collected and lyophilized to yield pure c[Gly-Tyr-Leu-Gln-Asn-Glu]-Gly-Leu-Gly-NH₂ **1** (444 mg) as a white powder with 99.7% purity (HPLC area-%, HPLC method cf. Example 1). No dimer was present in pure **1.** MS (m/z): 931.0

### Example 3 a-g (Optimization of coupling reagents):

c[Gly-Tyr-Ile-Gln-Asn-Glu]-Gly-Leu-Gly-NH₂ (1)

In an analogous manner to Example 2, the cyclizations were performed employing the coupling reagents as listed in Table 2.

**Table 2:**

| Example | Coupling reagent | Yield in reaction mixture (%) | Purity in reaction mixture (HPLC area-%) | Ratio of **1**/dimer |
|---|---|---|---|---|
| 3a | PyBOP | 63 | 54.9 | 13.4 |
| 3b | PyAOP | 63 | 52.7 | 23.2 |
| 3c | HBTU | 45 | 51.0 | 12.4 |
| 3d | HATU | 51 | 47.1 | 10.6 |
| 3e | HCTU | 55 | 10.6 | 13.8 |
| 3f | COMU | 26 | 11.5 | 28.0 |
| 3g | DMTMM | 17 | 18.5 | 34.9 |

### Example 4 a-g (Optimization of solvents):

c[Gly-Tyr-Ile-Gln-Asn-Glu]-Gly-Leu-Gly-NH₂ (1)

In an analogous manner to Example 2, the cyclizations were performed employing the solvents as listed in Table 3.

**Table 3:**

| Example | Solvent (Concentration) | Yield in reaction mixture (%) | Purity in reaction mixture (HPLC area-%) | Ratio of **1**/dimer |
|---|---|---|---|---|
| 4a | NEP (5 mM) | 63 | 60.3 | 30.4 |
| 4b | NEP (80 mM) | 63 | 54.9 | 13.4 |
| 4c | NMP (80 mM) | 44 | 46.9 | 13.3 |
| 4d | DMSO (80 mM) | 51 | 52.8 | 13.8 |
| 4e | DMF (80 mM) | 35 | 44.8 | 8.9 |
| 4f | DMI (80 mM) | 38 | 44.5 | 11.3 |
| 4g | DMPU (80 mM) | 35 | 45.3 | 17.7 |

### Example 5 a-g (Optimization of bases):

c[Gly-Tyr-Ile-Gln-Asn-Glu]-Gly-Leu-Gly-NH₂ (**1**)

In an analogous manner to Example 2, the cyclizations were performed employing the bases as listed in Table 4.

**Table 4:**

| Example | Base | Yield in reaction mixture (%) | Purity in reaction mixture (HPLC area-%) | Ratio of **1**/dimer |
|---|---|---|---|---|
| 5a | Imidazole | 63 | 55.7 | 10.6 |
| 5b | NMM | 68 | 57.7 | 22.0 |
| 5c | DABCO | 47 | 46.4 | 15.4 |
| 5d | DMAP | 43 | 47.8 | 13.6 |
| 5e | DIPEA | 63 | 54.9 | 13.4 |
| 5f | DBU | 25 | 27.0 | 19.4 |
| 5g | NMM, 0°C | 66 | 54.5 | 20.0 |

### Example 6 a-d (Comparison of the resin loading / amino acid equivalent):

c[Gly-Tyr-Ile-Gln-Asn-Glu]-Pro-Leu-Gly-NH₂ (**2**)

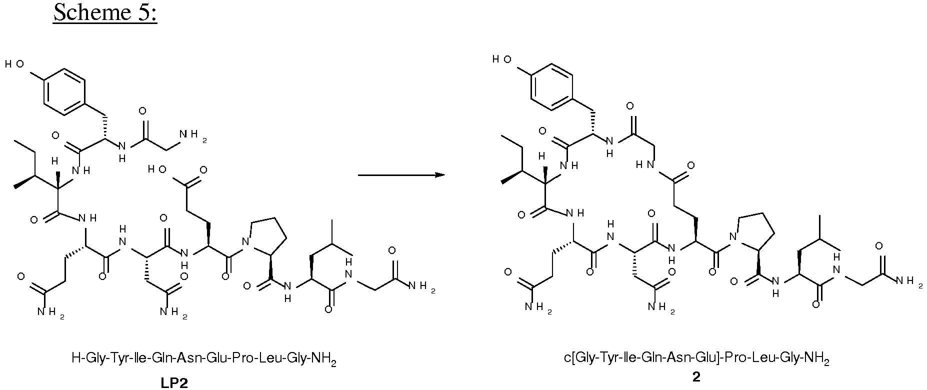

In an analogous manner to Example 2, pure cyclic peptide **2** was synthesized employing the Fmoc-AA-acids: Fmoc-Gly-OH, Fmoc-Leu-OH, Fmoc-Pro-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH
Scale of synthesis: 9.60 mmol (load: see example 6a-d; resin 30.0 g)
Yield: 40% (after purification)
Purity: 98.2% (HPLC area-%, HPLC method cf. example 1)
Retention time: 29.8 min (HPLC method cf. Example 1)
MS (m/z): 971.5 (M+H)⁺

Purity and yield of the linear peptide intermediate **LP2** (H-Gly-Tyr-Ile-Gln-Asn-Glu-Pro-Leu-Gly-NH₂) was determined employing the resin loadings / amino acid equivalents as listed in Table 5.

**Table 5:**

| Example | Loading of resin (mmol/g) | Amino acid (eq.) | Purity of crude **LP2** (HPLC area-%) | Yield of crude **LP2** (%) |
|---|---|---|---|---|
| 6a | 0.32 | 4 | 79.0 | 90 |
| 6b | 0.55 | 4 | 83.3 | 116 |
| 6c | 0.55 | 2 | 78.5 | 108 |
| 6d | 0.96 | 4 | 82.7 | 96 |

### Example 7

c[Gly-Tyr-Ile-Gln-Asn-Glu]-Pro-Leu-Gly-NH₂ (**2**)

Example 7 was performed in an analogous manner to Example 2, with the exception that the cyclizations were performed employing N-methylmorpholine as base.

### a) Fmoc-Cleavage:

A SPPS reactor (250 mL; peptide synthesizer CS536XT ex CSBio) was charged with PL-Rink resin (load. 0.55 mmol/g, 10.0 g, 5.50 mmol) and 20% piperidine in DMF (100 mL). The mixture was then stirred at 25 °C for 10 min. After draining the solvent, another portion of 20% piperidine in DMF (100 mL) was added and the mixture was stirred at 25 °C for 30 min. After draining the solvent, the resultant resin was washed with DMF (8 x 100 mL) to yield deFmoc-PL-Rink-resin.

### b) Coupling of Fmoc-AA-derivatives:

To deFmoc-PL-Rink-resin, a solution of Fmoc-Gly-OH in 0.35M HOBt/DMF (64.0 mL, 22.4 mmol), 0.92M DIC in DMF (32.0 mL, 29.4 mmol) and 10% pyridine in DMF (32.0 mL, 39.6 mmol) were added and stirred at 25 °C for 3 h. After draining the solvent, the resultant resin was washed with DMF (4 x 100 mL) to yield Fmoc-Gly-resin.

Fmoc-Cleavage and Fmoc-AA-derivative coupling steps were repeated 8 times employing instead of Fmoc-Gly-OH, the following Fmoc-amino acid-derivatives: Fmoc-Leu-OH, Fmoc-Pro-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH to yield Fmoc-Gly-Tyr(tBu)-Ile-Gln(Trt)-Asn(Trt)-Glu(tBu)-Pro-Leu-Gly-resin. A sample was cleaved from the resin (vehicle below) to confirm the correct mass. MS (m/z): 1211.1 (M+H)⁺

### c) Fmoc-Cleavage:

Fmoc-Cleavage of the terminal Gly was conducted as described above. After draining the solvent, the resultant resin was washed with DMF (8 x 100 mL), CH₂Cl₂ (3 x 100 mL) and MeOH (3 x 100 mL). The resin was dried under 10 mbar at 25 °C for 1 day to afford to yield H-Gly-Tyr(tBu)-Ile-Gln(Trt)-Asn(Trt)-Glu(tBu)-Pro-Leu-Gly-resin (18.6 g). A sample was cleaved from the resin (vehicle below) to confirm the correct mass. MS (m/z): 989.7 (M+H)⁺

### d) Global deprotection and resin cleavage:

To a precooled (10-15 °C) solution of triisopropylsilane (3.00 mL) in TFA (48.0 mL) and water (12.0 mL), H-Gly-Tyr(tBu)-Ile-Gln(Trt)-Asn(Trt)-Glu(tBu)-Pro-Leu-Gly-resin (6.00 g) was added and stirred at 25 °C for 3 h. The resin was filtered off and the filtrate was concentrated in vacuo. The residue was added to MeTHF (120 mL) and the mixture was stirred at 25 °C for 15 h. The mixture was filtered and the cake was washed with MeTHF (60.0 mL) followed by drying to afford H-Gly-Tyr-Ile-Gln-Asn-Glu-Pro-Leu-Gly-NH₂ **LP2** (1.84 g) as a white solid with 87.3% purity (HPLC area-%, HPLC method cf. Example 1). Retention time: 23.9 min (HPLC method cf. Example 1); MS (m/z): 989.7 (M+H)⁺

### e) Cyclization in solution:

To a mixture of H-Gly-Tyr-Ile-Gln-Asn-Glu-Pro-Leu-Gly-NH₂ **LP2** (300 mg) in N-ethylpyrrolidone (3.60 mL) and NMM (0.167 mL, 1.52 mmol) was added PyBOP (237 mg, 0.455 mmol) and stirred at 25 °C for 1 h. To complete conversion, another portion of PyBOP (47.4 mg, 0.0910 mmol) was added and stirred at the same temperature for 1 h. The resultant mixture was added to a solution of MTBE (24.0 mL) and MeTHF (6.00 mL), and then stirred at 25 °C for 15 h. The mixture was filtered and the cake was washed with MTBE (15.0 mL). The cake was dissolved in water/MeCN (10/1, 3.3 mL) and filtered off undissolved materials. The filtrate was lyophilized to afford crude c[Gly-Tyr-Leu-Gln-Asn-Glu]-Pro-Leu-Gly-NH₂**2**(313 mg, assay 54.0 wt%, total 60% yield) as a white solid with 71.4% purity (HPLC area-%, HPLC method cf. Example 1).MS (m/z): 971.5 (M+H)⁺

### Example 8

c[Gly-Tyr-Ile-Gln-Asn-Glu]-Sar-Leu-Gly-NH₂ (**3**)

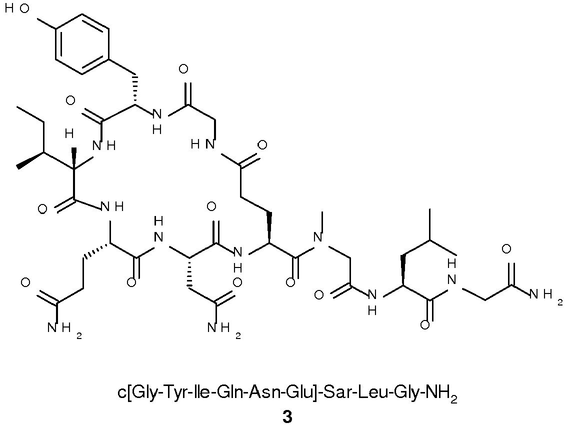

In an analogous manner to Example 2, pure cyclic peptide **3** was synthesized employing the Fmoc-AA-acids: Fmoc-Gly-OH, Fmoc-Leu-OH, Fmoc-Sar-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH
Scale of synthesis: 9.60 mmol (load. 0.32 mmol/g, resin 30.0 g)
Yield: 41% (after purification)
Purity: 98.9% (HPLC area-%, HPLC method cf. Example 1)
Retention time: 27.6 min (HPLC method cf. Example 1)
MS (m/z): 945.5 (M+H)⁺

### Example 9

c[Gly-Tyr-Ile-Gln-Asn-Glu]-Sar-Nle-Gly-NH₂ (**4**)

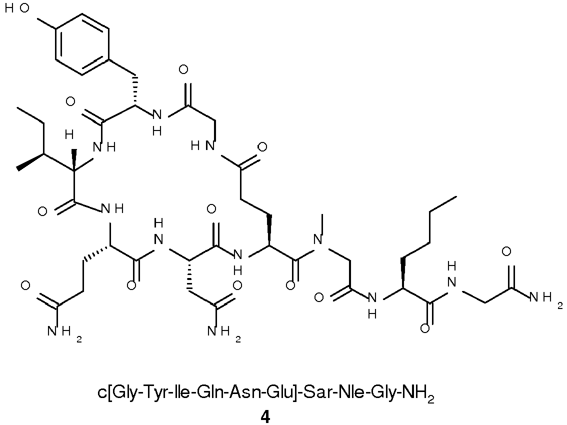

In an analogous manner to Example 2, pure cyclic peptide **4** was synthesized employing the Fmoc-AA-acids: Fmoc-Gly-OH, Fmoc-Nle-OH, Fmoc-Sar-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH
Scale of synthesis: 9.60 mmol (load. 0.32 mmol/g, resin 30.0 g)
Yield 41% (after purification)
Purity: 99.2% (HPLC area-%, HPLC method cf. Example 1)
Retention time: 25.9 min (HPLC method cf. Example 1)
MS (m/z): 945.5 (M+H)⁺

**Example 10**

c[Gly-Tyr-Ile-Gln-Asn-Glu]-trans-4-fluoro-Pro-Leu-Gly-NH₂ (**5**)

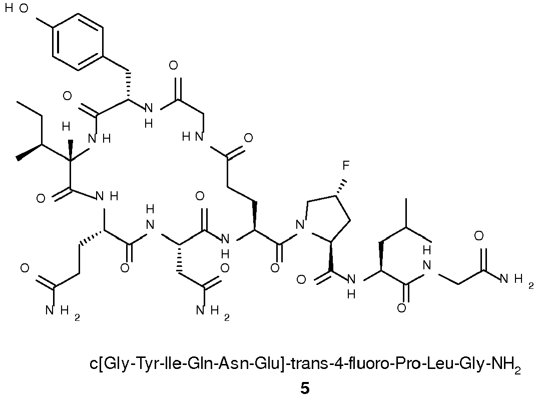

In an analogous manner to Example 2, pure cyclic peptide **5** was synthesized employing the Fmoc-AA-acids: Fmoc-Gly-OH, Fmoc-Leu-OH, Fmoc-trans-4-fluoro-Pro-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH
Scale of synthesis: 9.60 mmol (load. 0.32 mmol/g, resin 30.0 g)
Yield: 39% (after purification)
Purity: 98.8% purity (HPLC area-%, HPLC method cf. Example 1)
Retention time: 25.7 min (HPLC method cf. Example 1)
MS (m/z): 988.5 (M+H)⁺

### Example 11

c[Gly-Tyr-Ile-Gln-Asn-Glu]-trans-4-hydroxy-Pro-Leu-Gly-NH₂ (**6**)

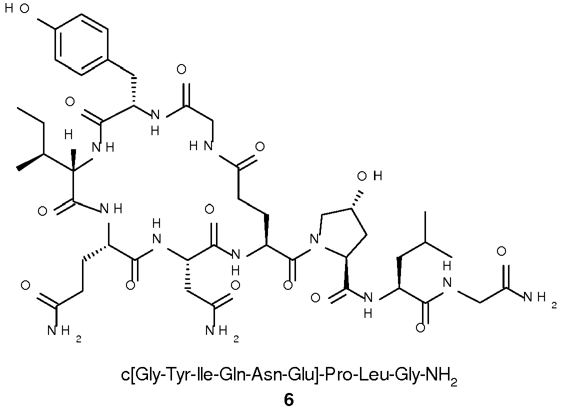

In an analogous manner to Example 2, pure cyclic peptide **6** was synthesized employing the Fmoc-AA-acids: Fmoc-Gly-OH, Fmoc-Leu-OH, Fmoc-trans-4-tertbutoxy-Pro-OH, Fmoc-Glu(tBu)-OH, Fmoc-Asn(Trt)-OH, Fmoc-Gln(Trt)-OH, Fmoc-Ile-OH, Fmoc-Tyr(tBu)-OH, Fmoc-Gly-OH
Scale of synthesis: 9.60 mmol (load. 0.32 mmol/g, resin 30.0 g)
Yield: 22% (after purification)
Purity: 98.7% purity (HPLC area-%, HPLC method cf. Example 1)
Retention time: 23.3 min (HPLC method cf. Example 1)
MS (m/z): 987.5 (M+H)⁺.

### Example 12 (Solid phase cyclization)

c[Gly-Tyr-Ile-Gln-Asn-Glu]-trans-4-fluoro-Pro-Leu-Gly-NH₂**_** **(5)**

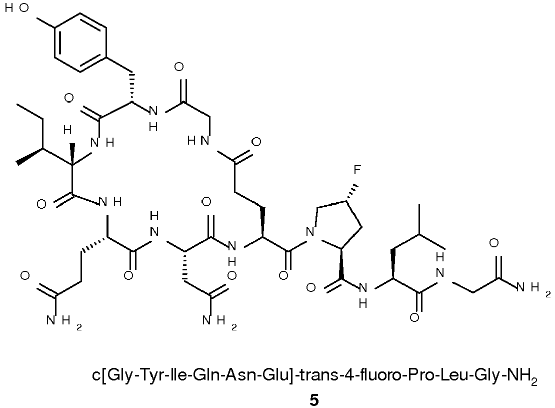

In an analogous manner to Example 1 employing a CS536XT peptide synthesizer from CSBio, pure cyclic peptide **5** was synthesized employing the Fmoc-AA-acids: Fmoc-Gly-OH, Fmoc-Leu-OH, Fmoc-trans-4-fluoro-Pro-OH, Fmoc-Glu(OAll)-OH, Fmoc-Asn(Trt)-OH, Fmoc-Gln(Trt)-OH, Fmoc-Ile-OH, Fmoc-Tyr(tBu)-OH, Fmoc-Gly-OH. Throughout the entire synthesis, for Fmoc-cleavage 10% 4-methyl-piperidine in DMF instead of 20% piperidine in DMF was used, and all amino acid couplings in the linear sequence were conducted employing HOPy instead of HOBt. In the final PyBOP promoted cyclization on resin step, 4-methylmorpholine instead of DIPEA was used as base and the cyclization was run in DMF instead of NEP as solvent. The preparative HPLC purification of crude c[Gly-Tyr-Ile-Gln-Asn-Glu]-trans-4-fluoro-Pro-Leu-Gly-NH₂ was conducted on a Kromasil-C18-100 column (250 x 4.6 mm, 10 um particle size, A: 20 mM NH4OAc pH5, B: MeCN; flow: 1 mL/min; isocratic 90/10 (A/B) for 1 min, gradient from 90/10 (A/B) to 80/20 (A/B) within 1 min, gradient from 80/20 (A/B) to 75/25 (A/B) within 10 min, gradient from 75/25 (A/B) to 10/90 (A/B) within 1 min, gradient from 10/90 (A/B) to for 5 min, gradient from 10/90 (A/B) to 90/10 (A/B) within 0.1 min, isocratic 90/10 (A/B) for 6.9 min. The collected fractions were diluted with water (1:1) and concentrated / desalted by loading on a conditioned (water/ACN 90/10) Kromasil C18-100-10 column (250 x 4.6 mm) and eluated afterwards with water/ACN (1:1). The collected fractions (UV 280 nm, threshold 1000mAu) were rotatory evaporated to remove ACN and lyophilized afterwards to yield the pure peptide as a white lyo product
Scale of synthesis: 5.50 mmol (loading 0.55 mmol/g, resin 10.0 g)
Yield: 34% (after purification)
Purity: 98.8% purity (HPLC area-%, HPLC method cf. Example 1)
Retention time: 25.3 min (HPLC method cf. Example 1)
MS (m/z): 989.5 (M+H)⁺

### Example 13 (Solid phase cyclization)

c[Gly-Tyr-Ile-Gln-Asn-Glu]-trans-4-fluoro-Pro-Leu-Gly-NH₂_ (**5**)

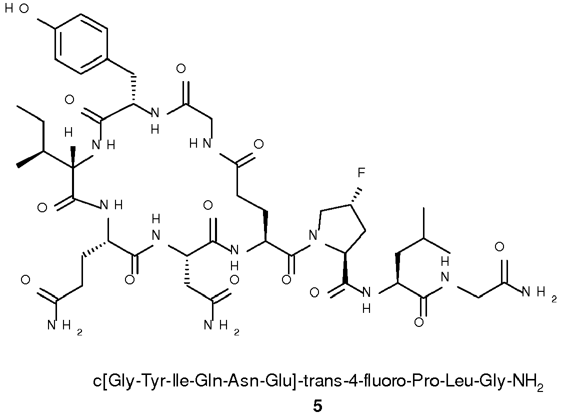

In an analogous manner to Example 13, pure cyclic peptide **5** was synthesized employing HOBt instead of HOPy throughout the entire synthesis of the linear peptide on resin.
Scale of synthesis: 5.50 mmol (loading 0.55 mmol/g, resin 10.0 g)
Yield: 25% (after purification)

## Claims

1. Process for the preparation of Oxytocin analogues of the formula I 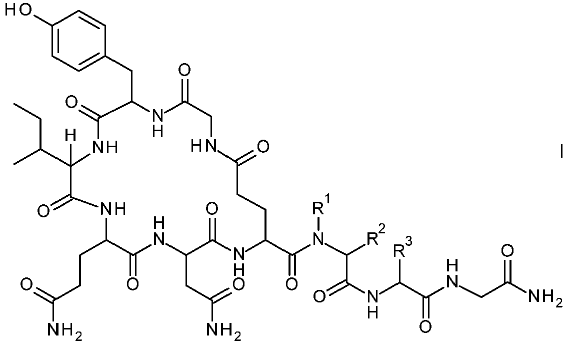 wherein
R¹ is hydrogen or C₁₋₇-alkyl and
R² is hydrogen or C₁₋₇-alkyl; or
R¹ and R² together with the nitrogen and the carbon atom to which they are attached form a 5-membered heterocyle which is optionally substituted with hydroxy or halogen;
R³ is C₁₋₇-alkyl
and of its corresponding enantiomers and/ or optical isomers thereof, comprising treating a resin bound peptide precursor of the formula II 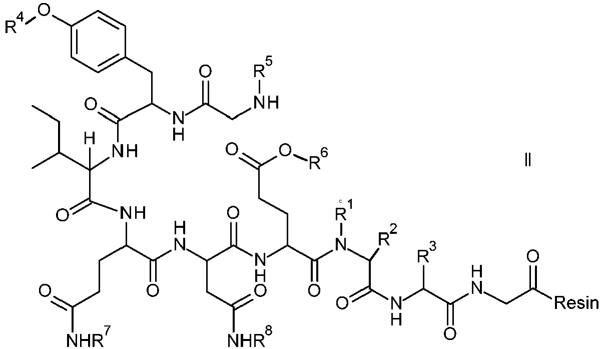 wherein
R¹, R² and R³ are as above and
R⁴ is a hydroxy protecting group;
R⁵ is Fmoc;
R⁶ is t-butyl, 1-adamantyl, or phenylisopropyl;
R⁷ is an amide protecting group; and
R⁸ is an amide protecting group
and its corresponding enantiomers and/ or optical isomers thereof,
according to the method:
b¹) the Fmoc group R⁵ is cleaved with a solution of piperidine or 4-methyl-piperidine in a suitable organic solvent;
b²) global deprotection and cleavage from the resin is effected in the presence of trifluoroacetic acid and a suitable scavenger such as thioanisole, anisole, phenol, triisopropylsilane, triethylsilane, ethanedithiol or dithiothreitol;
b³) ring cyclization is effected in solution using a cyclization agent selected from benzotriazol-1-yl-oxytripyrrolidinophosphonium hexafluorophosphate (PyBOP), (7-azabenzotriazol-1-yloxy)tripyrrolidinophosphonium hexafluorophosphate (PyAOP), N,N,N',N'-tetramethyl-O-(1H-benzotriazol-1-yl)uranium hexafluorophosphate (HBTU), 1-[bis(dimethylamino)methylene]-1H-1,2,3-triazolo[4,5-b]pyridinium 3-oxid hexafluorophosphate (HATU), O-(6-chlorobenzotriazol-1-yl)-N,N,N',N'-tetramethyluronium hexafluorophosphate (HCTU), (1-cyano-2-ethoxy-2-oxoethylidenaminooxy)dimethylamino-morpholino-carbenium hexafluorophosphate (COMU), 2-hydroxy-pyridine (HOPy) or 4-(4,6-dimethoxy-1,3,5-triazin-2-yl)-4-methylmorpholinium chloride (DMTMM) in the presence of an organic amine base selected from pyridine, imidazole, N,N-diisopropylethyl amine, triethylamine, N-methylmorpholine, N,N-dimethyl-4-aminopyridine, 1,8-Diazabicyclo[5.4.0]undec-7-ene or 1,4-diazabicyclo[2.2.2]octane;
b⁴) optionally the oxytocin analogue of formula I so obtained is isolated and purified.

2. Process of claim 1, wherein the Oxytocin analogue has the formula Ia 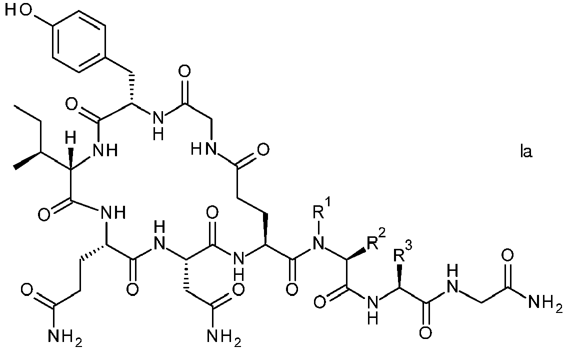 wherein R¹, R² and R³ are as above and wherein the resin bound peptide precursor of the formula II has the formula 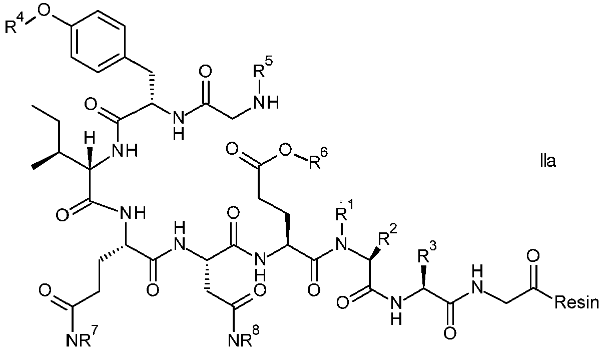 wherein R¹, R², R³, R⁴, R⁵, R⁶, R⁷ and R⁸ are as above.

3. Process of claims 1 or 2, wherein
R¹ is hydrogen or C₁₋₄-alkyl and
R² is hydrogen or C₁₋₄-alkyl; or
R¹ and R² together with the nitrogen and the carbon atom to which they are attached form a pyrrolidine ring of proline which is optionally substituted with hydroxy or halogen;
R³ stands for n-butyl or i-butyl;
R⁴ is t-butyl, allyl, trityl, 2-chlorotrityl, t-butyloxycarbonyl, t-butyldiphenylsilyl or t-butyldimethylsilyl;
R⁵ is Fmoc;
R⁶ is yl, 1-adamantyl, phenylisopropyl or t-butyl;
R⁷ is trityl, 2-chlorotrityl, 4-methyltrityl; and
R⁸ is trityl, 2-chlorotrityl, 4-methyltrityl.

4. Process of any of claims 1 to 3, wherein the resin bound peptide precursor of the formula II is prepared on the resin by repeated Fmoc cleavage and coupling of the respective Fmoc protected amino acids.

5. Process of any of claims 1 to 4, wherein the resin is a 4-[(2,4-Dimethoxyphenyl)Fmoc-aminomethyl]phenoxyacetamido methyl resin.

6. Process of claim 1 for the preparation of Oxytocin analogues of the formula Ia 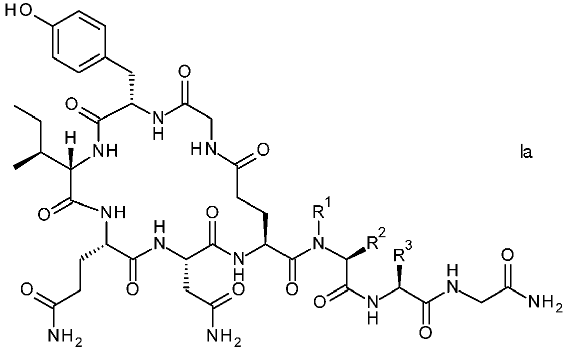 wherein
R¹ is hydrogen or C₁₋₄-alkyl and
R² is hydrogen or C₁₋₄-alkyl; or
R¹ and R² together with the nitrogen and the carbon atom to which they are attached form a pyrrolidine ring of proline which is optionally substituted with hydroxy or halogen;
R³ is n-butyl or i-butyl
and of its corresponding enantiomers and/ or optical isomers thereof.

## Patentansprüche

1. Verfahren zur Herstellung von Oxytocin-Analoga der Formel I 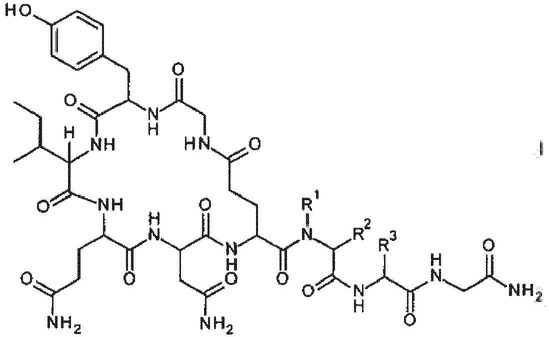 wobei
R¹ Wasserstoff oder C₁₋₇-Alkyl ist und
R² Wasserstoff oder C₁₋₇-Alkyl ist; oder
R¹ und R² zusammen mit dem Stickstoff- und dem Kohlenstoffatom, an die sie angebunden sind, einen 5-gliedrigen Heterocyclus bilden, der optional mit Hydroxy oder Halogen substituiert ist;
R³ C₁₋₇-Alkyl ist
und seiner entsprechenden Enantiomere und/oder optischen Isomere davon, umfassend das
Behandeln eines harzgebundenen Peptidvorläufers der Formel II 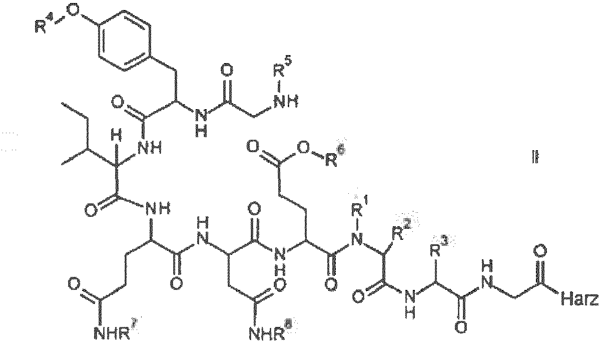 wobei
R¹, R² und R³ wie vorstehend sind und
R⁴ eine Hydroxyschutzgruppe ist;
R⁵ Fmoc ist;
R⁶ t-Butyl, 1-Adamantyl oder Phenylisopropyl ist;
R⁷ eine Amidschutzgruppe ist; und
R⁸ eine Amidschutzgruppe ist
und deren entsprechende Enantiomere und/oder optische Isomere davon,
nach dem Verfahren:
b¹) die Fmoc-Gruppe R⁵ wird mit einer Lösung aus Piperidin oder 4-Methylpiperidin in einem geeigneten organischen Lösungsmittel gespalten;
b²) Globale Entschützung und Abspaltung von dem Harz wird in der Gegenwart von Trifluoressigsäure und einem geeigneten Fänger wie Thioanisol, Anisol, Phenol, Triisopropylsilan, Triethylsilan, Ethandithiol oder Dithiotreitol ausgeführt;
b³) Ringcyclisierung wird in Lösung unter Verwendung eines Cyclisierungsmittels, das ausgewählt ist aus Benzotriazol-1-yl-oxytripyrrolidinophosphoniumhexafluorphosphat (PyBOP), (7-Azabenzotriazol-1-yloxy)tripyrrolidinophosphoniumhexafluorphosphat (PyAOP), N,N,N',N'-Tetramethyl-O-(1H-benzotriazol-1-yl)uraniumhexafluorphosphat (HBTU), 1-[bis(Dimethylamino)methylen]-1H-1,2,3-triazolo[4,5-b]pyridinium-3-oxidhexafluorphosphat (HATU), O-(6-Chlorbenzotriazol-1-yl)-N,N,N',N'-tetramethyluroniumhexafluorphosphat (HCTU), (1-Cyano-2-ethoxy-2-oxoethylidenaminooxy)dimethylaminomorpholinocarbeniumhexafluorphosphat (COMU), 2-Hydroxypyridin (HOPy) oder 4-(4,6-Dimethoxy-1,3,5-triazin-2-yl)-4-methylmorpholiniumchlorid (DMTMM) in der Gegenwart einer organischen Aminbase ausgewählt aus Pyridin, Imidazol, N,N-Diisopropylethylamin, Triethylamin, N-Methylmorpholin, N,N-Dimethyl-4-aminopyridin, 1,8-Diazabicyclo[5.4.0]undec-7-en oder 1,4-Diazabicyclo[2.2.2]octan;
b⁴) optional wird das so erhaltene Oxytocin-Analog der Formel I isoliert und gereinigt.

2. Verfahren nach Anspruch 1, wobei das Oxytocin-Analog die Formel Ia aufweist 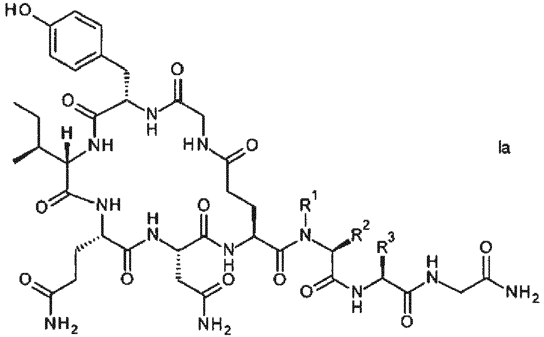 wobei R¹, R² und R³ wie vorstehend sind und wobei der harzgebundene Peptidvorläufer der Formel II die folgende Formel aufweist 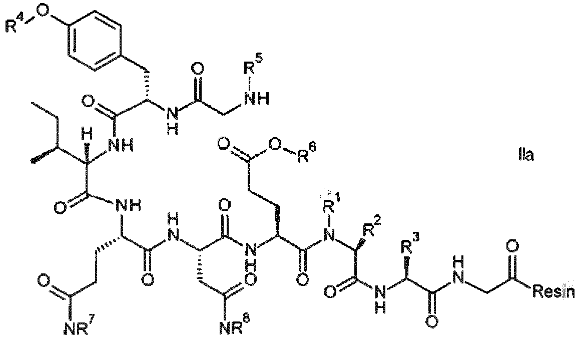 wobei R¹, R², R³, R⁴, R⁵, R⁶, R⁷ und R⁸ wie vorstehend sind.

3. Verfahren nach den Ansprüchen 1 oder 2, wobei
R¹ Wasserstoff oder C₁₋₄-Alkyl ist und
R² Wasserstoff oder C₁₋₄-Alkyl ist; oder
R¹ und R² zusammen mit dem Stickstoff- und dem Kohlenstoffatom, an die sie gebunden sind, einen Pyrrolidinring von Prolin bilden, der optional mit Hydroxy oder Halogen substituiert ist;
R³ für n-Butyl oder i-Butyl steht;
R⁴ t-Butyl, Allyl, Trityl, 2-Chlortrityl, t-Butyloxycarbonyl, t-Butyldiphenylsilyl oder t-Butyldimethylsilyl ist;
R⁵ Fmoc ist;
R⁶ yl, 1-Adamantyl, Phenylisopropyl oder t-Butyl ist;
R⁷ Trityl, 2-Chlortrityl, 4-Methyltrityl ist; und
R⁸ Trityl, 2-Chlortrityl, 4-Methyltrityl ist.

4. Verfahren nach einem der Ansprüche 1 bis 3, wobei der harzgebundene Peptidvorläufer der Formel II auf dem Harz durch wiederholte Fmoc-Spaltung und Kupplung der entsprechenden Fmoc-geschützten Aminosäuren hergestellt wird.

5. Verfahren nach einem der Ansprüche 1 bis 4, wobei das Harz ein 4-[(2,4-Dimethoxyphenyl)Fmoc-Aminomethyl]phenoxyacetamidomethylharz ist.

6. Verfahren nach Anspruch 1 zur Herstellung von Oxytocin-Analoga der Formel Ia 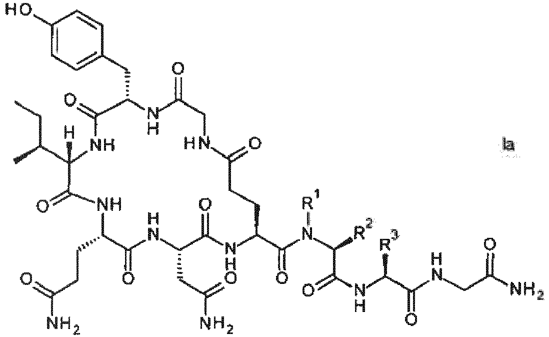 wobei
R¹ Wasserstoff oder C₁₋₄-Alkyl ist und
R² Wasserstoff oder C₁₋₄-Alkyl ist; oder
R¹ und R² zusammen mit dem Stickstoff- und dem Kohlenstoffatom, an die sie gebunden sind, einen Pyrrolidinring von Prolin bilden, der optional mit Hydroxy oder Halogen substituiert ist;
R³ n-Butyl oder i-Butyl ist
und seiner entsprechenden Enantiomere und/oder optischen Isomere davon.

## Revendications

1. Procédé pour la préparation d'analogues de l'oxytocine de formule I 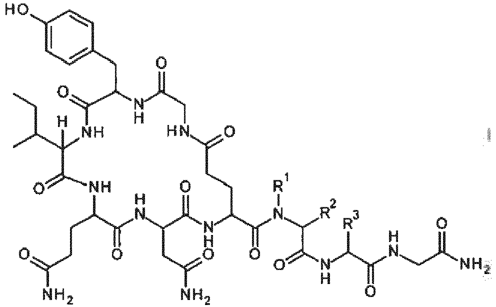 dans laquelle
R¹ représente un hydrogène ou un alkyle en C₁₋₇ et
R² représente un hydrogène ou un alkyle en C₁₋₇ ; ou
R¹ et R² forment, conjointement avec l'atome d'azote et l'atome de carbone auxquels ils sont liés, un hétérocycle à 5 chaînons qui est éventuellement substitué par un hydroxy ou un halogène ;
R³ représente un alkyle en C₁₋₇
et de ses énantiomères correspondants et/ou isomères optiques, comprenant le traitement d'un précurseur peptidique lié à une résine de formule II 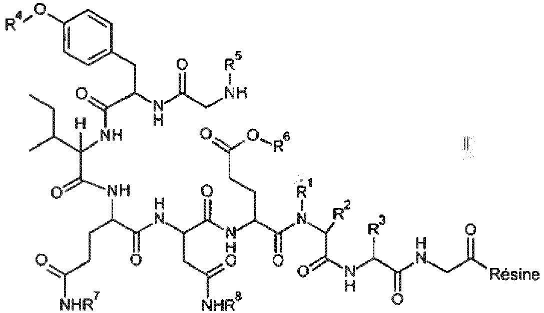 dans laquelle
R¹, R² et R³ sont tels que ci-dessus et
R⁴ représente un groupe protecteur d'hydroxy ;
R⁵ représente Fmoc ;
R⁶ représente un t-butyle, un 1-adamantyle ou un phénylisopropyle ;
R⁷ représente un groupe protecteur d'amide ; et
R⁸ représente un groupe protecteur d'amide
et de ses énantiomères correspondants et/ou isomères optiques,
selon le procédé :
b¹) le groupe Fmoc R⁵ est clivé avec une solution de pipéridine ou de 4-méthyl-pipéridine dans un solvant organique approprié ;
b²) une déprotection globale et un clivage de la résine sont mis en oeuvre en présence d'acide trifluoroacétique et d'un désactivateur approprié tel que le thioanisole, l'anisole, le phénol, le triisopropylsilane, le triéthylsilane, l'éthanedithiol ou le dithiothréitol ;
b³) une cyclisation est mise en oeuvre en solution en utilisant un agent de cyclisation choisi parmi l'hexafluorophosphate de benzotriazol-1-yl-oxytripyrrolidinophosphonium (PyBOP), l'hexafluorophosphate de (7-azabenzotriazol-1-yloxy)tripyrrolidinophosphonium (PyAOP), l'hexafluorophosphate de N,N,N',N'-tétraméthyl-O-(1H-benzotriazol-1-yl)uranium (HBTU), le 3-oxydo-hexafluorophosphate de 1-[bis(diméthylamino)méthylène]-1H-1,2,3-triazolo[4,5-b]pyridinium (HATU),
l'hexafluorophosphate de O-(6-chlorobenzotriazol-1-yl)-N,N,N',N'-tétraméthyluronium (HCTU), l'hexafluorophosphate de (1-cyano-2-éthoxy-2-oxoéthylidènaminooxy)diméthylamino-morpholino-carbénium (COMU), la 2-hydroxy-pyridine (HOPy) ou le chlorure de 4-(4,6-diméthoxy-1,3,5-triazin-2-yl)-4-méthylmorpholinium (DMTMM) en présence d'une base amine organique choisie parmi la pyridine, l'imidazole, la N,N-diisopropyléthylamine, la triéthylamine, la N-méthylmorpholine, la N,N-diméthyl-4-aminopyridine, le 1,8-diazabicyclo[5.4.0]undéc-7-ène ou le 1,4-diazabicyclo[2.2.2]octane ;
b⁴) l'analogue de l'oxytocine de formule 1 ainsi obtenu est éventuellement isolé et purifié.

2. Procédé selon la revendication 1, dans lequel l'analogue de l'oxytocine a la formule la 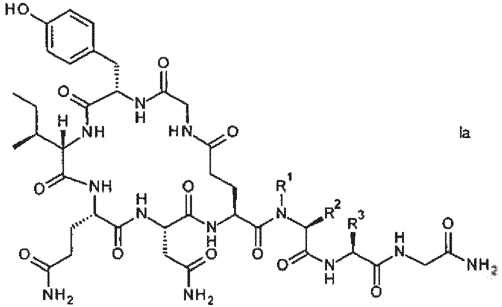 dans laquelle R¹, R² et R³ sont tels que ci-dessus, et dans lequel le précurseur peptidique lié à une résine de formule II a la formule 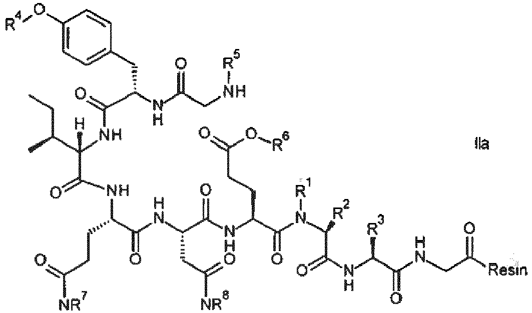 dans laquelle R¹, R², R³, R⁴, R⁵, R⁶, R⁷ et R⁸ sont tels que ci-dessus.

3. Procédé selon les revendications 1 ou 2, dans lequel
R¹ représente un hydrogène ou un alkyle en C₁₋₄ et
R² représente un hydrogène ou un alkyle en C₁₋₄ ; ou
R¹ et R² forment, conjointement avec l'atome d'azote et l'atome de carbone auxquels ils sont liés, un noyau pyrrolidine de proline qui est éventuellement substitué par un hydroxy ou un halogène ;
R³ représente un n-butyle ou un i-butyle ;
R⁴ représente un t-butyle, un allyle, un trityle, un 2-chlorotrityle, un t-butyloxycarbonyle, un t-butyldiphénylsilyle ou un t-butyldiméthylsilyle ;
R⁵ représente Fmoc ;
R⁶ représente yl, un 1-adamantyle, un phénylisopropyle ou un t-butyle ;
R⁷ représente un trityle, un 2-chlorotrityle, un 4-méthyltrityle ; et
R⁸ représente un trityle, un 2-chlorotrityle, un 4-méthyltrityle.

4. Procédé selon l'une quelconque des revendications 1 à 3, dans lequel le précurseur peptidique lié à une résine de formule II est préparé sur la résine par la répétition du clivage du Fmoc et du couplage des acides aminés protégés par le Fmoc respectifs.

5. Procédé selon l'une quelconque des revendications 1 à 4, dans lequel la résine est une résine 4-[(2,4-diméthoxyphényl)Fmoc-aminométhyl]phénoxyacétamido-méthyle.

6. Procédé selon la revendication 1 pour la préparation d'analogues de l'oxytocine de formule la 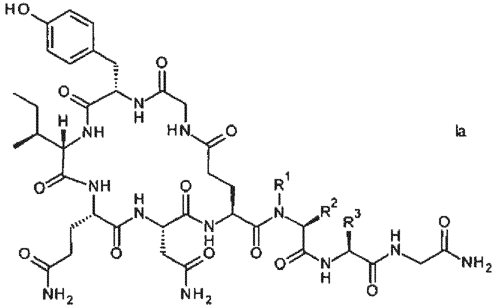 dans laquelle
R¹ représente un hydrogène ou un alkyle en C₁₋₄ et
R² représente un hydrogène ou un alkyle en C₁₋₄ ; ou
R¹ et R² forment, conjointement avec l'atome d'azote et l'atome de carbone auxquels ils sont liés, un noyau pyrrolidine de proline qui est éventuellement substitué par un hydroxy ou un halogène ;
R³ représente un n-butyle ou un i-butyle
et de ses énantiomères correspondants et/ou isomères optiques.
